# EUROPEAN PATENT APPLICATION

(11) **EP 4 613 839 A1**
(43) Date of publication of application: **10.09.2025**
(21) Application number: 23886304.7
(22) Date of filing: 02.11.2023
(51) Int. Cl.: C12M 1/12, A61L 2/18, C12N 1/00

(54) **METHOD OF PROMOTING PERACETIC ACID DECOMPOSITION USING METAL COMPOUND, AND METHOD OF CULTURING MICROORGANISMS USING SAME**

(30) Priority: 03.11.2022 KR 20220145352
(71) Applicant: N-Cell Co.,Ltd, Chungcheongbuk-do 27721 (KR)
(72) Inventor: CHO, Changho, Seoul 06146 (KR)
(74) Representative: Gille Hrabal Partnerschaftsgesellschaft mbB Patentanwälte
(86) International application number: PCT/KR2023/017353
(87) International publication number: WO 2024/096609

(57) **Abstract**

The present invention relates to a method for decomposing peracetic acid using a metal compound and a method for culturing microorganisms using the decomposition method. The cultivation of microorganisms using the method of the present invention allows an effective removal of the peracetic acid used in a culture medium for sterilization.

## Description

### Technical field

The present invention relates to a method for decomposing peracetic acid using metal compounds and a method for culturing microorganisms using the aforementioned decomposition method.

### Background Art

In the bioindustry, when attempting to mass-produce microbial raw materials and useful metabolites derived from microorganisms, sterilization of the culture medium and reactors is of significant importance.

Conventionally, an autoclave, a sterilization method using high temperature and high pressure, has primarily been used to sterilize microbial culture media and reactors.

The sterilization of culture media and reactors in an autoclave is, however, problematic in that the sugar component of the culture medium reacts with one of the other components of the medium, proteins (peptone or yeast extract) at high temperature to cause the generation of toxic substances such as hydroxymethyl furfural (HMF) capable of hindering the growth of microorganisms and that the reactors are required to be made of materials resistant to heat and pressure like metals or glass.

A method of filtration is used to overcome the above problems and limitations. But, the filtration method causes an inconvenience of requiring a separate sterilization of the incubator and a risk of clogging and re-contamination during the filtration process and involves a limited size of the filter and a high cost, thus resulting in poor commercial availability.

In order to solve this problem, a sterilization method using peracetic acid (PAA) was devised as disclosed in Korean Patent Publication No. 10-2015-0097295. This publication discloses a method for sterilizing photobioreactors using peracetic acid when cultivating microalgae that require light in pure culture. However, the prior document does not disclose any method for sterilizing a medium containing sugars and nitrogen sources (such as peptone, yeast extract, or whey). Moreover, in the method for sterilizing photobioreactors and media using peracetic acid, when the medium contains sugars and nitrogen sources, a high concentration of peracetic acid is required for complete sterilization. Particularly, when sterilizing media that include nitrogen sources, nitrogen oxides (NO₃⁻) are generated by peracetic acid, and the produced nitrogen oxides bind with iron ions, inhibiting the catalytic reaction needed for peracetic acid decomposition. Consequently, relying solely on natural decomposition of peracetic acid and the catalytic reaction by iron and HEPES takes more than a month to decompose the peracetic acid reactant.

Meanwhile, as disclosed in Korean Patent Publication No. 10-2020-0083909, when sterilizing by adding iron ions, alkali metal hydroxides, EDTA, and sugar to a peracetic acid mixture and causing a reaction to decompose the peracetic acid mixture into acetic acid, water, and oxygen, a long time is required for the decomposition of peracetic acid. Additionally, the acetic acid produced as a decomposition byproduct inhibits or kills certain microorganisms, presenting a technical limitation.

Accordingly, the inventors have completed the present invention by developing a method for promoting the decomposition of peracetic acid using metal compounds and a method for culturing microorganisms utilizing this decomposition method, in order to address the issues of prior techniques.

### Disclosure

### Technical problems

An object of the present invention is to provide a method for decomposing peracetic acid, comprising the steps of: mixing peracetic acid with water; and adding iron ions, an alkali metal hydroxide, ethylenediaminetetraacetic acid (EDTA), a sugar, and a metal compound to the mixture.

Another object of the present invention is to provide a method for sterilizing a medium, which comprises the steps of: adding peracetic acid to a medium containing sugars and nitrogen sources to achieve sterilization; and adding iron ions, an alkali metal hydroxide, ethylenediaminetetraacetic acid (EDTA), and a metal compound to the sterilized medium as a promoter for the decomposition of peracetic acid.

Another object of the present invention is to provide a method for culturing microorganisms, comprising the steps of: adding a medium sterilized by the above method to a bioreactor; and inoculating the added medium with microorganisms and culturing them in pure culture.

### Technical Solution

In order to achieve the above objects, the present invention provides a method for decomposing peracetic acid, comprising the steps of: mixing peracetic acid with water; and adding iron ions, an alkali metal hydroxide, ethylenediaminetetraacetic acid (EDTA), a sugar, and a metal compound to the mixture.

The present invention also provides a method for sterilizing a medium, comprising the steps of: adding peracetic acid to a medium containing sugars and nitrogen sources to sterilize it; and adding iron ions, an alkali metal hydroxide, ethylenediaminetetraacetic acid (EDTA), and a metal compound to the sterilized medium as a promoter for peracetic acid decomposition.

The present invention further provides a method for culturing microorganisms, comprising the steps of: adding a medium sterilized by the above method to a bioreactor; and inoculating the added medium with microorganisms and culturing them in pure culture.

### Advantageous Effects

The present invention relates to a method for decomposing peracetic acid using metal compounds and a method for culturing microorganisms using the aforementioned decomposition method. By using the method of the present invention, peracetic acid present in the medium can be decomposed at a significantly rapid rate within a few minutes, thereby allowing for the cultivation of microorganisms in the sterilized medium.

### Brief description of drawings

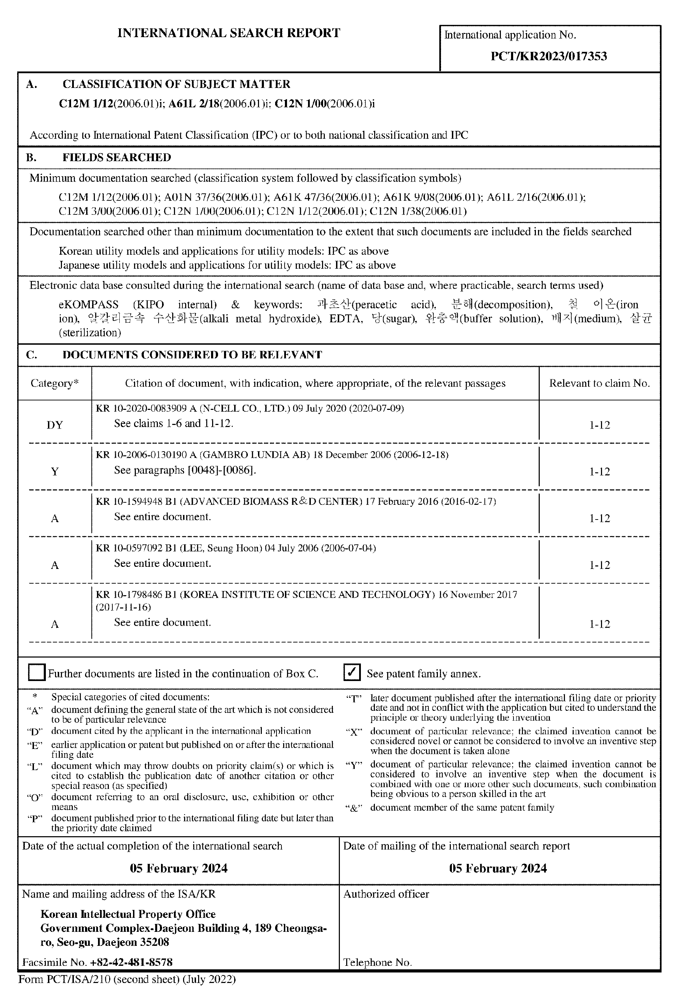

Fig. 1 shows the growth status of *Euglena gracilis* based on the concentration of acetic acid added to the medium. The culture conditions in each culture bottle, from left to right, correspond to acetic acid concentrations of 0%, 0.05%, 0.15%, 0.1%, 0.2%, and 0.25% (w/v), representing the control and Comparative Examples 2 to 6.
Fig. 2 shows the growth status of *Euglena gracilis* based on the concentration of sodium acetate added to the medium. The culture conditions in each culture bottle, from left to right, correspond to sodium acetate concentrations of 0%, 0.05%, 0.1%, 0.15%, 0.2%, and 0.25% (w/v), representing the control and Examples 11 to 15.
Fig. 3 shows the growth status of *Euglena gracilis* based on the concentration of acetic acid added to the medium (Comparative Examples 2 to 6) and the growth status of the same species based on the concentration of sodium acetate added to the medium (Examples 11 to 15). The Y-axis represents absorbance, where SA denotes sodium acetate and SS denotes acetic acid.

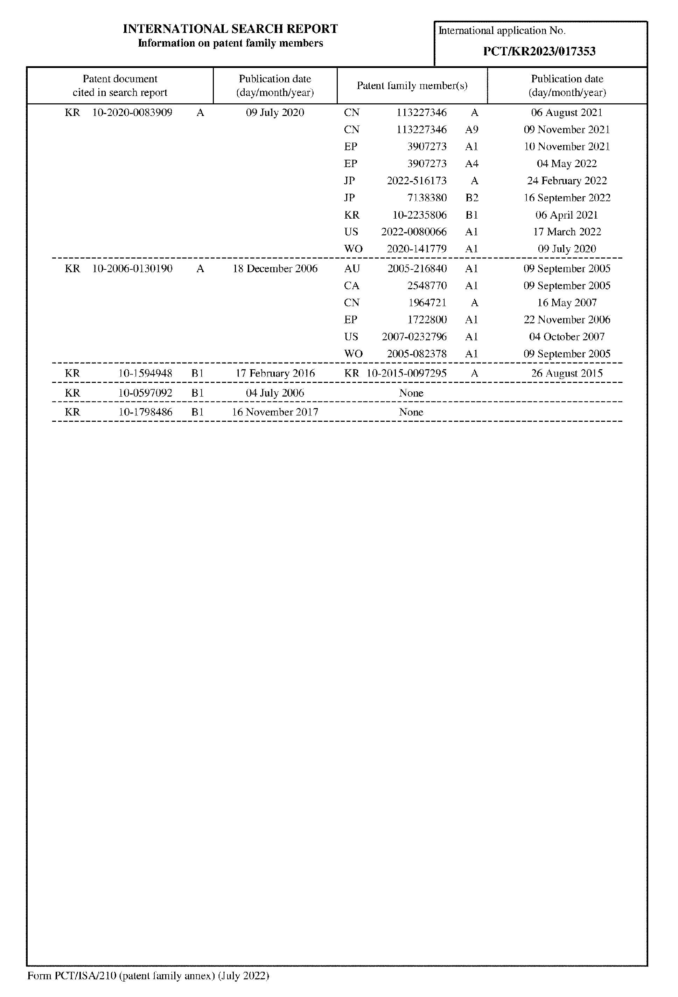

Fig. 4 shows the growth status of *Euglena gracilis* based on the concentration of sodium bicarbonate added to the medium (Examples 16 to 21 and Comparative Example 7), with the results from Table 5 presented in a graphical format. Fig. 5 is a perspective view showing the internal configuration of the cultivation module shown in Fig. 2.

### Mode for Invention

Hereinafter, the present invention will be described in detail with reference to the accompanying drawings and embodiments.

However, the following embodiments are provided merely as examples of the present invention, and specific descriptions of well-known technologies or configurations may be omitted if it is determined that such descriptions could unnecessarily obscure the essence of the present invention. This omission does not limit the present invention in any way. The present invention is capable of various modifications and applications within the scope of the claims described below and their equivalents as interpreted.

Throughout this specification, when a component is described as being 'connected' (or 'joined,' 'in contact,' or 'coupled') to another component, this includes both cases where it is 'directly connected' and cases where it is 'indirectly connected' with other members interposed in between. Additionally, when a component is described as 'including' another component, it means that, unless explicitly stated otherwise, other components may also be present and are not necessarily excluded.

The terminology used in this specification is employed merely to describe particular embodiments and is not intended to limit the scope of the present invention. Singular expressions include plural expressions unless the context clearly indicates otherwise. **In** this specification, terms such as 'comprise' or 'have' specify the presence of stated features, numbers, steps, actions, components, parts, or combinations thereof but do not preclude the possibility of the presence or addition of one or more other features, numbers, steps, actions, components, parts, or combinations thereof.

Additionally, the terminology used in this specification is chosen to appropriately describe the preferred embodiments of the present invention, and it may vary depending on the intent of the user, operator, or convention within the field to which the invention belongs. Therefore, definitions of these terms should be determined based on the overall content of this specification. Throughout the specification, when a part is described as 'including' a component, this means that other components may also be included, unless explicitly stated otherwise.

The term 'sterilization,' as used in the present invention, refers to the killing of microbial vegetative cells and spores.

The term 'metal compound' as used in the present invention may refer to a compound containing metal ions, such as sodium, calcium, potassium, which are capable of binding with acetic acid.

The term 'alkali metal' as used in the present invention may refer to elements belonging to Group 1 of the periodic table, and specifically may include lithium (Li), sodium (Na), and potassium (K), excluding hydrogen.

The term 'peracetic acid mixture' as used in the present invention may refer to a mixture created by combining peracetic acid with water, and in the context of the present invention, the peracetic acid mixture may include peracetic acid, acetic acid, and hydrogen peroxide.

The term 'peracetic acid reactant' as used in the present invention may refer to a mixture obtained by adding iron ions, an alkali metal hydroxide, ethylenediaminetetraacetic acid (EDTA), sugar, and a metal compound to the peracetic acid mixture. In the context of the present invention, the peracetic acid reactant may include hydrogen peroxide, acetic acid, or sodium acetate (CH₃COONa). Additionally, depending on the added metal compound, the peracetic acid reactant may include sodium acetate (CH₃COONa), calcium acetate ((CH₃COO)₂Ca), or potassium acetate (CH₃COOK), but is not limited thereto.

The term 'medium' as used in the present invention, also referred to as culture medium or culture solution, refers to a substance designed to maintain a nutrient environment similar to the natural conditions in which microorganisms generally survive and propagate, with the objective of microbial growth or cultivation. It includes both liquid and solid substances supplemented with all necessary nutrients for growth. The cultivation of microorganisms in culture media depends on various essential factors, such as the selection of essential nutrients, gases (such as oxygen) needed for growth, moisture, pH, and temperature. Important nutrient sources may include carbon, nitrogen, inorganic phosphate, sulfur, trace metals, water, and vitamins.

As an aspect for achieving the above technical objectives, the present invention provides a method for decomposing peracetic acid, comprising the steps of:
mixing peracetic acid with water; and adding iron ions, an alkali metal hydroxide, ethylenediaminetetraacetic acid (EDTA), sugar, and a metal compound to the mixture,
wherein the metal compound includes one or more selected from the group consisting of sodium bicarbonate (NaHCO₃), disodium hydrogen phosphate (Na₂HPO₄), calcium carbonate (CaCO₃), calcium lactate pentahydrate (Ca(CH₃CHOHCOO)₂·5H₂O), and dipotassium hydrogen phosphate (K₂HPO₄).

In the method for decomposing peracetic acid according to the present invention, the iron ions may be Fe²⁺ ions or Fe³⁺ ions, and the iron ions may be derived from salts containing Fe ions such as FeCl₃, FeCl₂, and/or FeSO₄.

The alkali metal hydroxide may be LiOH, NaOH, or KOH, but is not limited thereto.

The sugar may be dextrose, glucose, sucrose, and/or waste sugar (molasses), but is not limited thereto.

The metal compound may be characterized by including one or more selected from the group consisting of sodium bicarbonate (NaHCO₃), disodium hydrogen phosphate (Na₂HPO₄), calcium carbonate (CaCO₃), calcium lactate pentahydrate (Ca(CH₃CHOHCOO)₂·5H₂O), and dipotassium hydrogen phosphate (K₂HPO₄).

The concentration of the metal compound is characterized by being 0.005%(w/v) or higher, preferably 0.025%(w/v) or higher, more preferably 0.05%(w/v) or higher, and most preferably 0.08%(w/v) or higher.

The method for decomposing peracetic acid in the present invention may be characterized by the ability to significantly accelerate the overall peracetic acid decomposition reaction by removing acetic acid, which inhibits microbial growth, from the peracetic acid reactant. In one embodiment of the present invention, to remove acetic acid included in the peracetic acid reactant, one or more compounds selected from the group consisting of sodium bicarbonate (NaHCO₃), disodium hydrogen phosphate (Na₂HPO₄), calcium carbonate (CaCO₃), calcium lactate pentahydrate (Ca(CH₃CHOHCOO)₂·5H₂O), and dipotassium hydrogen phosphate (K₂HPO₄) were used. However, the metal compound may include compounds containing ions, such as sodium, potassium, and/or calcium, capable of binding with acetic acid, and the scope of the invention is not limited thereto.

The step of mixing peracetic acid with water in the method for decomposing peracetic acid according to the present invention may involve mixing peracetic acid and water at room temperature to produce a peracetic acid mixture, wherein the peracetic acid mixture may include hydrogen peroxide and acetic acid.

1) CH₃CO₃H (peracetic acid) + H₂O → H₂O₂ (hydrogen peroxide) + CH₃CO₂H (acetic acid)

The hydrogen peroxide produced from the reaction of the peracetic acid and water is susceptible to the Fenton reaction driven by the iron ion and decomposed into acetic acid, water and oxygen according to the following chemical equations.

2) 2CH₃CO₃H → 2CH₃CO₂H + O₂

3) 2H₂O₂ → 2H₂O + O₂

At this time, the hydrogen peroxide can be used as an oxidizing agent, bleaching agent, and disinfectant, and in the context of the present invention, it can be used to sterilize the medium.

The step of adding iron ions, an alkali metal hydroxide, ethylenediaminetetraacetic acid (EDTA), sugar, and a metal compound to the peracetic acid mixture in the decomposition method of peracetic acid according to the present invention may be carried out at room temperature to produce a peracetic acid reactant. The peracetic acid reactant may include hydrogen peroxide, acetic acid, or sodium acetate (CH₃COONa). Additionally, depending on the added metal compound, the peracetic acid reactant may include sodium acetate (CH₃COONa), calcium acetate ((CH₃COO)₂Ca), or potassium acetate (CH₃COOK), but is not limited thereto.

The following example illustrates the generation of sodium acetate (CH₃COONa) by adding sodium bicarbonate (NaHCO₃) to acetic acid (CH₃COOH).

4-1) CH₃COOH+NaHCO₃→ CH₃COONa+CO₂+H₂O

4-2) 2CH₃COOH+Na2HPO₄→2CH₃COONa+H₃PO₄

The following example illustrates the generation of calcium acetate ((CH₃COO)₂Ca) by adding calcium carbonate (CaCO₃) to acetic acid (CH₃COOH).40: Temperature control unit

4-3) 2CH₃COOH+CaCO₃→(CH₃COO)₂Ca+CO₂+H₂O

4-4) 2CH₃COOH+Ca(CH₃CHOHCOO)₂·5H₂O+6O₂→(CH₃COO)₂Ca+6CO₂+6H₂O

The following example illustrates the generation of sodium acetate by adding sodium bicarbonate to acetic acid (CH₃COOH).

4-5) CH₃COOH+KH₂PO₄→CH₃COOK+H₂PO₄⁻

4-6) 2CH₃COOH+K₂HPO₄→2CH₃COOK+H₃PO₄

In the method for decomposing peracetic acid according to the present invention, a basic buffer solution may be used in place of, or in addition to, the alkali metal hydroxide. The basic buffer solution may be 4-(2-hydroxyethyl)-1-piperazineethanesulfonic acid (HEPES), but is not limited thereto.

In another aspect, the present invention provides a method for sterilizing a medium, comprising the steps of: adding peracetic acid to a medium containing sugars and nitrogen sources to sterilize it;
and adding iron ions, an alkali metal hydroxide, ethylenediaminetetraacetic acid (EDTA), and a metal compound to the sterilized medium as a promoter for peracetic acid decomposition.

The metal compound includes one or more selected from the group consisting of sodium bicarbonate (NaHCO₃), disodium hydrogen phosphate (Na₂HPO₄), calcium carbonate (CaCO₃), calcium lactate pentahydrate (Ca(CH₃CHOHCOO)₂·5H₂O), and dipotassium hydrogen phosphate (K₂HPO₄).

In the method for sterilizing a medium according to the present invention, specific details of the peracetic acid decomposition method may be appropriately applied without departing from the essence of the present invention.

The method for sterilizing a medium according to the present invention can decompose peracetic acid, the peracetic acid mixture, and the peracetic acid reactant present in the sterilized medium, thereby enabling the medium produced by this method to be used for microbial cultivation.

In the method for sterilizing a medium according to the present invention, the iron ions may be Fe²⁺ ions or Fe³⁺ ions, and the iron ions may be derived from salts containing Fe ions, such as FeCl₃, FeCl₂, and/or FeSO₄.

In the method for sterilizing a medium according to the present invention, the alkali metal hydroxide may be LiOH, NaOH, or KOH, but is not limited thereto.

In the method for sterilizing a medium according to the present invention, the sugar may be dextrose, glucose, sucrose, and/or waste sugar(molasses), but is not limited thereto.

In the method for sterilizing a medium according to the present invention, the concentration of the metal compound is characterized by being 0.005%(w/v) or higher, preferably 0.025%(w/v) or higher, more preferably 0.05%(w/v) or higher, and most preferably 0.08% (w/v) or higher.

In the method for sterilizing a medium according to the present invention, a basic buffer solution may be used in place of, or in addition to, the alkali metal hydroxide, and the basic buffer solution may be 4-(2-hydroxyethyl)-1-piperazineethanesulfonic acid (HEPES).

When the medium is sterilized using the method according to the present invention, it can be stored for an extended period without contamination, even in an open container, and without requiring autoclaving prior to neutralization.

In another aspect, the present invention provides a method for culturing microorganisms, comprising the steps of:
adding a medium sterilized by the above method to a bioreactor; and inoculating the added medium with microorganisms and culturing them in pure culture.

In the method for culturing microorganisms according to the present invention, specific details of the method for sterilizing the medium may be appropriately applied without departing from the essence of the present invention.

In the method for culturing microorganisms according to the present invention, the microorganisms may include *Aurantiochytrium* sp., *Schizochytrium* sp., *Chlorella* sp., *Synechocystis* sp., *Debaryomyces* sp., yeasts, lactic acid bacteria, actinomycetes, *Euglena, Mortierella,* filamentous fungi, and/or photosynthetic bacteria, but are not limited thereto.

The term 'microbial inoculation' may refer to the act of introducing microorganisms into an environment suitable for their growth, and may include methods such as the streak plate method, spread plate method, and pour plate method, but is not limited thereto.

The term 'pure culture' refers to the cultivation of microorganisms or similar organisms in a medium where only a single species is present. In this case, a method of confirming pure culture may involve verifying the formation of a single colony through the streak plate method.

According to one embodiment of the present invention, when sodium bicarbonate is added to the peracetic acid mixture, the rate of peracetic acid decomposition significantly increases, and it was confirmed that the decomposition rate gradually increases as the concentration of sodium bicarbonate increases (Examples 1 to 6 and Comparative Example 1).

Hereinafter, the present invention will be described in detail through embodiments. However, the following embodiments are provided to illustrate the present invention, and the scope of the present invention is not limited to these embodiments.

### Example 1. Confirmation of Peracetic Acid Decomposition Promotion by Sodium Bicarbonate

A microbial medium was prepared by adding 5 g/L of yeast extract and 20 g/L of dextrose to 1 L of distilled water, followed by the addition of peracetic acid (Dongmyung ONC, 160126) to achieve a peracetic acid solution concentration of 0.05% (v/v).

To the solution, 96 µM FeCl₃ (Sigma, 7705-08-0), 5.5 mM NaOH (Sigma, 1310-73-2), and 96 µM EDTA (Sigma, 60-00-4) were added, followed by the addition of sodium bicarbonate (NaHCO₃, Samchun Pure Chemical, S0343) to achieve a concentration of 0.05% (w/v).

The extent of peracetic acid decomposition was measured using the Peroxide Test (MQuant 1100810001), which detects peroxide, a decomposition byproduct of the peracetic acid solution.

### Comparative Example 1. Without Addition of Sodium Bicarbonate

The experiment was conducted in the same manner as in Example 1, except that sodium bicarbonate was not added.

### Examples 2 to 6. Variations in Sodium Bicarbonate Concentration

The experiment was conducted in the same manner as in Example 1, except that the concentration of sodium bicarbonate was adjusted to 0.1%(w/v) (Example 2), 0.15%(w/v) (Example 3), 0.2%(w/v) (Example 4), 0.25%(w/v) (Example 5), and 0.3%(w/v) (Example 6), respectively.

### Example 7. Confirmation of Peracetic Acid Decomposition Promotion by Disodium Hydrogen Phosphate

The experiment was conducted in the same manner as in Example 1, except that disodium hydrogen phosphate (Na₂HPO₄, Samchun Pure Chemical, S0893) was added at a concentration of 0.05%(w/v) instead of sodium bicarbonate.

### Example 8. Confirmation of Peracetic Acid Decomposition Promotion by Calcium Carbonate

The experiment was conducted in the same manner as in Example 1, except that calcium carbonate (CaCO₃, Samchun Pure Chemical, S0076) was added at a concentration of 0.05%(w/v) instead of sodium bicarbonate.

### Example 9. Confirmation of Peracetic Acid Decomposition Promotion by Calcium Lactate

The experiment was conducted in the same manner as in Example 1, except that calcium lactate (Ca(CH₃CHOHCOO)₂·5H₂O, Daejung Chemicals, 2513-4405) was added at a concentration of 0.05%(w/v) instead of sodium bicarbonate.

### Example 10. Confirmation of Peracetic Acid Decomposition Promotion by Dipotassium Hydrogen Phosphate

The experiment was conducted in the same manner as in Example 1, except that dipotassium hydrogen phosphate (K₂HPO₄, Samchun Pure Chemical, P1098) was added at a concentration of 0.05% (w/v) instead of sodium bicarbonate.

### Example 11. Confirmation of Microbial Growth Inhibition by Sodium Acetate

The following experiment was conducted to determine whether sodium acetate, a decomposition product of peracetic acid, inhibits microbial growth.

A nutrient medium for the cultivation of *Euglena gracilis* (prepared with triple-distilled water, KH₂PO₄, citrate, MgSO₄·3H₂O, vitamin B1, vitamin B6, vitamin B12, vitamin H, yeast extract, peptone, and dextrose) was autoclaved at 121°C for 15 minutes. Sodium acetate (CH₃COONa) was then added to the prepared nutrient medium at a concentration of 0.05% (w/v), and an inoculum of *Euglena gracilis* (KCTC AG40099) was introduced. The culture was incubated at 130 rpm, 28°C, and under light conditions of 100 µE photons/m²/s. Growth was measured by monitoring absorbance at a wavelength of 680 nm.

### Examples 12 to 15. Variations in Sodium Acetate Concentration

The experiment was conducted in the same manner as in Example 11, except that sodium acetate was added at concentrations of 0.1%(w/v) (Example 12), 0.15%(w/v) (Example 13), 0.2%(w/v) (Example 14), and 0.25%(w/v) (Example 15), respectively.

### Comparative Example 2. Confirmation of Microbial Growth Inhibition by Acetic Acid

The experiment was conducted in the same manner as in Example 11, except that acetic acid was added instead of sodium acetate.

### Comparative Examples 3 to 6. Variations in Acetic Acid Concentration

The experiment was conducted in the same manner as in Comparative Example 2, except that acetic acid was added to the prepared nutrient medium at concentrations of 0.1%(w/v) (Comparative Example 3), 0.15%(w/v) (Comparative Example 4), 0.2%(w/v) (Comparative Example 5), and 0.25%(w/v) (Comparative Example 6), instead of sodium acetate.

### Example 16. Confirmation of the Effect of Sodium Bicarbonate on Microbial Growth for Promoting Peracetic Acid Decomposition

The following experiment was conducted to determine the effect of sodium bicarbonate, added to the peracetic acid mixture, on microbial growth.

Specifically, a nutrient medium for the cultivation of *Euglena gracilis* (prepared with triple-distilled water, KH₂PO₄, citrate, MgSO₄·3H₂O, vitamin B1, vitamin B6, vitamin B12, vitamin H, yeast extract, peptone, and dextrose) was autoclaved at 121°C for 15 minutes, and then peracetic acid was added to the medium to achieve a concentration of 0.05%(w/v). Then, 96 µM FeCl₃ (Sigma, 7705-08-0), 5.5 mM NaOH (Sigma, 1310-73-2), and 96 µM EDTA (Sigma, 60-00-4) were added to the medium, followed by the addition of sodium bicarbonate (NaHCO₃) to achieve a concentration of 0.05%(w/v). After confirming the decomposition of the added peracetic acid using the Peroxide Test (MQuant 1100810001), *Euglena gracilis* was inoculated and cultured. The growth of *Euglena* was measured by monitoring absorbance at a wavelength of 680 nm.

### Comparative Example 7. Without Addition of Sodium Bicarbonate

The experiment was conducted in the same manner as in Example 16, except that sodium bicarbonate was not added.

### Examples 17 to 21. Variations in Sodium Bicarbonate Concentration

The experiment was conducted in the same manner as in Example 16, except that the concentration of sodium bicarbonate was adjusted to 0.1%(w/v) (Example 17), 0.15%(w/v) (Example 18), 0.20%(w/v) (Example 19), 0.25%(w/v) (Example 20), and 0.3%(w/v) (Example 21), respectively.

### Experimental Example 1. Confirmation of Peracetic Acid Decomposition Promotion Effect by Varying Sodium Bicarbonate Concentrations

To confirm the effect of sodium bicarbonate and its concentration on peracetic acid decomposition, the experiment was conducted in the same manner as in Examples 1 to 6 and Comparative Example 1.

As a result, it was confirmed that (i) the addition of the metal compound sodium bicarbonate (Examples 1 to 6) significantly increased the rate of peracetic acid decomposition compared to when sodium bicarbonate was not added (Comparative Example 1) (Table 1), and (ii) increasing the concentration of sodium bicarbonate from 0.05% to 0.25% led to an increase in the rate of peracetic acid decomposition.

Specifically, when sodium bicarbonate was added at 0.2%(w/v) or higher (Examples 4 to 6), the decomposition of peracetic acid was completed within minutes, whereas in the absence of sodium bicarbonate (Comparative Example 1), complete decomposition of peracetic acid took 48 hours (Table 1). Even when sodium bicarbonate was added at 0.05%(w/v) (Example 1), peracetic acid was fully decomposed within 3 hours. Therefore, compared to Comparative Example 1, which required over 24 hours for decomposition, the decomposition time was reduced to less than 1/8, demonstrating a significant increase in the decomposition rate.

**[Table 1]**

| Time | Comparative Experiment | Example 1 | Example 2 | Example 3 | Example 4 | Example 5 | Example 6 |
|---|---|---|---|---|---|---|---|
| | PAA | PAANHC (0.05) | PAANHC (0.1) | PAANHC (0.15) | PAANHC (0.2) | PAANHC (0.25) | PAANHC (0.3) |
| 10 min | >100 | >100 | >100 | 50 | 10 | 0 | 0 |
| 1h | >100 | >100 | 30 | 10 | 0 | 0 | 0 |
| 2h | >100 | >100 | 0 | 0 | 0 | 0 | 0 |
| 3h | >100 | 0 | 0 | 0 | 0 | 0 | 0 |
| 12h | >100 | 0 | 0 | 0 | 0 | 0 | 0 |
| 24h | 30 | 0 | 0 | 0 | 0 | 0 | 0 |
| 48h | 0 | 0 | 0 | 0 | 0 | 0 | 0 |

| | | | | | | | |
|---|---|---|---|---|---|---|---|
| -NHC: NaHCO₃ -The numbers in Tables 1 to 5 indicate the concentration of remaining peroxide, with '>100' indicating that more than 100 mg/L of peroxide remains. -The numbers in parentheses in Tables 1 to 5 represent the concentration of NaHCO₃ in %(w/v). | | | | | | | |

### Experimental Example 2: Confirmation of Peracetic Acid Decomposition Promotion Effect by Type of Metal Compound

To confirm the effect of metal compounds on peracetic acid decomposition, the experiment was conducted in the same manner as in Example 1, Examples 7 to 9, and Comparative Example 1.

As a result, when the metal compound sodium bicarbonate (NaHCO₃) was added (Example 1), peracetic acid decomposed rapidly within 1 hour. When Na₂HPO₄ was added (Example 7), the decomposition rate was also significantly increased, with complete decomposition occurring within 12 hours. Similarly, when calcium carbonate was added (Example 8), complete decomposition was achieved within 1 hour, comparable to sodium bicarbonate. In addition, when calcium lactate or dipotassium hydrogen phosphate was added, complete decomposition occurred within 24 hours, demonstrating an increased decomposition rate compared to the case without metal compounds (Examples 9 and 10) (Table 2).

**[Table 2]**

| time[H] | Comparative Example 1 | Example 1 | Example 7 | Example 8 | Example 9 | Example 10 |
|---|---|---|---|---|---|---|
| | PAA | PAANHC | PAANa₂HPO₄ | PAACaCO₃ | PAACa(CH₃C HOHCOO)₂ · 5H2O | PAAK₂HPO₄ |
| 0 | >100 | >100 | >100 | >100 | >100 | >100 |
| 3 | >100 | 0 | >100 | 0 | >100 | >100 |
| 6 | >100 | 0 | 50 | 0 | >100 | >100 |
| 12 | >100 | 0 | 0 | 0 | >100 | 50 |
| 18 | >100 | 0 | 0 | 0 | 30 | 10 |
| 24 | 30 | 0 | 0 | 0 | 0 | 0 |
| 30 | 10 | 0 | 0 | 0 | 0 | 0 |
| 36 | 0 | 0 | 0 | 0 | 0 | 0 |

| | | | | | | |
|---|---|---|---|---|---|---|
| -NHC: NaHCO₃ -The numbers in Tables 1 to 5 indicate the concentration of remaining peroxide; for example, '>100' means that more than 100 mg/L of peroxide remains. -The concentrations of PAA and the metal compounds in Table 2 are each 0.05%(w/v). | | | | | | |

### Experimental Example 3: Confirmation of Microbial Growth Inhibition by Acetic Acid or Sodium Acetate

To confirm the effect of acetic acid (CH₃COOH), a decomposition product of peracetic acid, and sodium acetate (CH₃COONa), the final decomposition product, on microbial growth, the experiment was conducted in the same manner as in Examples 11 to 15 and Comparative Examples 2 to 6.

As a result, in Comparative Example 2, where the concentration of acetic acid was 0.05%(w/v), there was no inhibitory effect on the growth of *Euglena gracilis.* However, starting from concentrations of 0.1%(w/v) or higher (Comparative Examples 3 to 6), growth inhibition of *Euglena gracilis* was observed (Fig. 1).

Meanwhile, in the groups with added sodium acetate (Examples 11 to 15), *Euglena* was observed to grow well under all conditions (Fig. 2), confirming that sodium acetate does not inhibit the growth of *Euglena.*

The numbers in Tables 3 and 4 represent the absorbance (AU, 680 nm) of the *Euglena gracilis* culture solution, and the results from Tables 3 and 4 are presented graphically in Fig. 3.

**[Table 3]**

| Cultivation Time [Days] | Comparative Example 2 | Comparative Example 3 | Comparative Example 4 | Comparative Example 5 | Comparative Example 6 |
|---|---|---|---|---|---|
| | 0.05 %(w/v) | 0.1 %(w/v) | 0.15 %(w/v) | 0.2 %(w/v) | 0.25 %(w/v) |
| 0 Day | 1.3738 | 1.8372 | 1.831 | 1.686 | 1.7554 |
| 1 Day | 2.1945 | 1.712 | 1.598 | 1.5025 | 1.5195 |
| 2 Days | 3.929 | - | - | - | - |
| 4 Days | 10.34 | - | - | - | - |
| 5 Days | 11.063 | - | - | - | - |

**[Table 4]**

| Cultivation Time [Days] | Example 11 | Example 12 | Example 13 | Example 14 | Example 15 |
|---|---|---|---|---|---|
| | 0.05 %(w/v) | 0.1 % (w/v) | 0.15 %(w/v) | 0.2 %(w/v) | 0.25 %(w/v) |
| 0 Day | 1.33 | 1.316 | 1.3882 | 1.3602 | 1.4034 |
| 1 Day | 2.2345 | 2.1825 | 2.1605 | 2.1565 | 2.1945 |
| 2 Days | 3.898 | 3.969 | 3.861 | 3.709 | 3.803 |
| 4 Days | 8.511 | 7.739 | 7.52 | 7.624 | 7.369 |
| 5 Days | 9.852 | 8.442 | 8.561 | 8.665 | 8.405 |

### Experimental Example 4: Confirmation of Microbial Growth Inhibition by Sodium Bicarbonate

To confirm the effect of sodium bicarbonate, used to decompose peracetic acid and its decomposition products to the final products, on the growth of *Euglena gracilis,* the experiment was conducted in the same manner as in Comparative Example 7 and Examples 16 to 21.

As a result, high growth of *Euglena* was observed in all experimental groups, including Comparative Example 7, which contained only peracetic acid, and Examples 16 to 21, which included both peracetic acid and sodium bicarbonate. This confirmed that sodium bicarbonate, used to decompose peracetic acid and its decomposition products, does not specifically affect the growth of *Euglena gracilis* (Table 5 and Fig. 4).

**[Table 5]**

| Cultivati on Time [Days] | Comparative Example 7 | Example 16 | Example 17 | Example 18 | Example 19 | Example 20 | Example 21 |
|---|---|---|---|---|---|---|---|
| | PAA | PAA NaHCO₃ 0.05(w/v) % | PAA NaHCO₃ 0.1 (w/v) % | PAA NaHCO₃0. 15(w/v)% | PAANaHC O₃0.2(w/v) % | PAA NaHCO₃0.2 5(w/v)% | PAA NaHCO₃0.3( w/v)% |
| 0 Day | 2.992 | 3.114 | 3.036 | 3.025 | 2.768 | 2.908 | 2.707 |
| 1 Day | 3.515 | 3.887 | 4.136 | 4.305 | 4.284 | 4.176 | 4.285 |
| 2 Days | 4.693 | 5.513 | 6.963 | 6.867 | 6.424 | 6.279 | 5.957 |
| 3 Days | 5.782 | 6.518 | 7.935 | 8.714 | 8.578 | 8.187 | 7.72 |
| 4 Days | 6.773 | 8.177 | 9.025 | 9.259 | 9.339 | 9.493 | 9.051 |
| 5 Days | 8.414 | 9.029 | 9.917 | 9.691 | 9.619 | 10.671 | 9.848 |

| | | | | | | | |
|---|---|---|---|---|---|---|---|
| -The concentration of PAA in Table 5 is 0.05%(w/v). | | | | | | | |

## Claims

1. A method for decomposing peracetic acid, comprising:
adding iron ions, an alkali metal hydroxide, ethylenediaminetetraacetic acid (EDTA), sugar, and a metal compound to the mixture,
wherein the metal compound comprises one or more selected from the group consisting of sodium bicarbonate (NaHCO₃), disodium hydrogen phosphate (Na₂HPO₄), calcium carbonate (CaCO₃), calcium lactate pentahydrate (Ca(CH₃CHOHCOO)₂·5H₂O), and dipotassium hydrogen phosphate (K₂HPO₄).

2. The method according to claim 1, wherein the iron ion is Fe²⁺ or Fe³⁺.

3. The method according to claim 1, wherein the sugar is glucose, sucrose, or waste sugar.

4. The method according to claim 3, wherein the concentration of the metal compound is 0.005%(w/v) or higher.

5. The method according to claim 1, wherein a basic buffer solution is further added.

6. A method for sterilizing a medium, comprising:
(1) adding peracetic acid to a medium containing sugars and nitrogen sources to sterilize the medium; and
(2) adding iron ions, an alkali metal hydroxide, ethylenediaminetetraacetic acid (EDTA), and a metal compound as a promoter for peracetic acid decomposition to the sterilized medium, wherein the metal compound comprises one or more selected from the group consisting of sodium bicarbonate (NaHCO₃), disodium hydrogen phosphate (Na₂HPO₄), calcium carbonate (CaCO₃), calcium lactate pentahydrate (Ca(CH₃CHOHCOO)₂·5H₂O), and dipotassium hydrogen phosphate (K₂HPO₄).

7. The method according to claim 6, wherein the iron ion is Fe²⁺ or Fe³⁺.

8. The method according to claim 6, wherein the sugar is glucose, sucrose, or waste sugar.

9. The method according to claim 6, wherein the concentration metal compound is 0.005%(w/v) or higher.

10. The method according to claim 6, wherein a basic buffer solution is further added.

11. A method for culturing microorganisms, comprising:
(1) adding a medium sterilized by the method of claim 6 to a bioreactor; and
(2) inoculating microorganisms into the added medium and conducting a pure cultivation.

12. The method according to claim 11, wherein the microorganisms include at least one selected from the group consisting of *Aurantiochytrium* sp., *Schizochytrium* sp., *Chlorella* sp., *Synechocystis* sp., *Debaryomyces* sp., Yeasts, Lactic acid bacteria, *Actinomycetes, Euglena, Mortierella* filamentous fungus, and photosynthetic bacteria.
